# EUROPÄISCHE PATENTSCHRIFT

(11) **EP 2 120 845 B1**
(45) Veröffentlichungstag und Bekanntmachung des Hinweises auf die Patenterteilung: **24.10.2012**
(21) Anmeldenummer: 07712282.8
(22) Anmeldetag: 21.02.2007
(51) Int. Cl.: A61K 8/02, A61K 8/04, A61K 8/73, A61K 8/85, A61Q 17/04, A61Q 19/00

(54) **NANOPARTIKEL ZUR VERWENDUNG IN KOSMETISCHEN UND DERMATOLOGISCHEN ZUBEREITUNGEN**
NANOPARTICLES FOR USE IN COSMETIC AND DERMATOLOGICAL FORMULATIONS
NANOPARTICULES À UTILISER DANS DES PRÉPARATIONS COSMÉTIQUES ET DERMATOLOGIQUES

(43) Veröffentlichungstag der Anmeldung: 25.11.2009
(73) Patentinhaber: ROVI Cosmetics International GmbH, 36381 Schlüchtern (DE)
(72) Erfinder: BLUME, Gabriele, 36396 Steinau A.d. Strasse (DE); TEICHMÜLLER, Dirk, 63589 Linsengericht (DE)
(74) Vertreter: WSL Patentanwälte Partnerschaftsgesellschaft
(86) Internationale Anmeldenummer: PCT/EP2007/051694
(87) Internationale Veröffentlichungsnummer: WO 2008/101546

(56) Entgegenhaltungen:
- WO-A-00/35415
- JP-A- 2000 143 533
- US-A1- 2001 010 824

## Beschreibung

Die vorliegende Erfindung betrifft kosmetische oder dermatologische Zubereitungen mit einem Wirkstoff zum Schutz oder zur Behandlung der Haut und/oder der Haare und mit einem Trägermaterial in oder an dem der Wirkstoff gebunden ist oder mit dem der Wirkstoff assoziiert ist, wobei das Trägermaterial einen Anteil an Chitosan aufweist.

Die menschliche Haut ist ein sehr großes Organ mit einer Fläche von annähernd 2 m², einem Gewicht von 3 kg und einer mittleren Gesamtdicke von ungefähr 2 mm. Der Haut kommt als Grenzorgan zwischen Organismus und Umwelt eine wichtige Bedeutung zu. Sie bietet Schutz gegenüber Verletzungen, ist für viele gasförmige und gelöste Stoffe schwer zu durchdringen und dient gleichzeitig auch als Barriere gegen das Eindringen von Mikroorganismen. Um diesen vielfältigen Funktionen gerecht werden zu können, hat die Haut einen Aufbau mit entsprechend komplexer Struktur. Hierfür sind in die Haut unter anderem verschiedene Anhangorgane, wie Haare, Nägel und Drüsen eingebettet. Eine besondere Bedeutung bei der Funktion als äußere Schutzschicht kommt allerdings der Epidermis zu. Die äußerste Schicht der Epidermis ist das Stratum Corneum (SC) und stellt die eigentliche penetrationshemmende Barriere der Haut dar.

Der Aufbau des Stratum Corneum wird modellhaft nach Barry et al. anhand des Ziegelstein-Mörtel-Prinzips beschrieben. Dabei stellen die zu 50 % aus dem Skleroprotein Keratin bestehenden Korneozyten die Ziegelsteine und die sie umgebende, hauptsächlich aus Sphingolipiden, Cholesterol und freien Fettsäuren bestehende Lipid-Wasser-Schicht den Mörtel bzw. die Lipidmatrix dar. Eine Überwindung dieser Schicht wie auch ihre Beeinflussung wird durch kosmetische, dermatologische und pharmazeutische Zubereitungen angestrebt.

Kosmetischen und dermatologischen Zubereitungen kommt insbesondere die Aufgabe zu, die beanspruchte Haut und ihre Anhanggebilde vor schädlichen Umwelteinflüssen, wie z.B. Sonne und Wind, Chemikalien und Mikroorganismen, zu schützen, aber auch vor dem Verlust körpereigener Stoffe zu bewahren. Eine Hautpflege ist gerade dann besonders notwendig, wenn das körpereigene Regenerationsvermögen der Haut nicht mehr ausreicht oder eine Belastung durch die Umwelteinflüsse eine verstärkte Hautalterung nach sich zieht.

Die chemische Zusammensetzung von kosmetischen, dermatologischen und pharmazeutischen Zubereitungen und das Zeitprofil der Freisetzung der in solchen Zubereitungen enthaltenen Wirkstoffe haben einen wesentlichen Einfluß auf die Struktur der Haut bzw. ihrer Anhanggebilde. Zweckmäßige Zubereitungen sind in der Lage, die Barriereeigenschaften der obersten Hautschichten vorteilhaft zu verbessern, die natürliche Funktion der Haut aufrecht zu erhalten, diese zu schützen und zu pflegen.

Zum Schutz der Haut und zu ihrer Pflege gibt es Zubereitungen wie Emulsionen (z.B. O/W, W/O, W/O/W etc.), Suspensionen, Salben, Cremes, Gele, Pasten etc. Von immer größerer Bedeutung ist es, daß solche Zubereitungen sehr gut verträglich sind.

Als besonders gut verträglich erwiesen sich Zubereitungen mit biologisch resorbierbaren und/oder biologisch abbaubaren Trägerstoffen. Solche Stoffe können vom menschlichen Körper beispielsweise über die Haut resorbiert werden und gegebenenfalls im Körper beispielsweise durch körpereigene Stoffwechselprozesse abgebaut werden. Ein Beispiel für einen biologisch abbaubaren Trägerstoff ist das Chitosan.

Aus DE 36 44 097 und DE 39 12 122 sind kosmetische Mittel zur Behandlung der Haare oder der Haut auf der Basis von Chitosan bekannt. Chitosan ist ein natürlich vorkommendes Polyaminosaccharid, welches sich vom Chitin ableitet. Es besteht aus β-1,4-glykosidisch verknüpften N-Acetylglucosaminresten. Chitosan und dessen Derivate haben ein gutes Haftvermögen auf der Haut und auf dem Haar. Häufig rufen Mittel mit Chitosan bzw. Chitosanderivaten jedoch ein unangenehmes Klebrigkeitsgefühl auf der Haut hervor. Außerdem sind die Transporteigenschaften von Chitosan enthaltenden Zubereitungen oft nicht optimal. Darüber hinaus läßt sich die Freisetzungskinetik im Hinblick auf den Wirkstoff in solchen Zubereitungen nicht in dem gewünschten Maße beeinflussen.

JP 2000 143533 offenbart Nanosphäre, die aus einem Kern aus Polylaktid-Glycolid Copolymer und Calcitonine umhüllt mit Chitosan besteht. Die Calcitonine sind Wirkstoffe, die transpulmonär verabreicht werden. US 2001/010824 offenbart bioabbaubare Nanopartikel enthaltend aktive Öle oder Wirkstoff-enthaltende Öle. In den Beispielen 16 bis 19 wird Polylaktid als umhüllendes bioabbaubares Polymer verwendet. WO 00/35415 offenbart nanopartikuläre Lichtschutzfilter, wobei die organischen Wirkstoffe mit Schutzkolloiden formuliert werden. In Beispiel 4 wird Chitosan als Schutzkolloid verwendet.

Es ist daher die Aufgabe der vorliegenden Erfindung kosmetische oder dermatologische Zubereitungen mit sehr gutem Anhaftungsverhalten an bzw. in der Haut und deren Anhangorganen, wie z.B. den Haaren, bereitzustellen, wobei das in den Zubereitungen verwendete Trägermaterial biologisch resorbierbar und/oder abbaubar sein soll und wobei die Zubereitungen gegenüber herkömmlichen Zubereitungen möglichst verbesserte Transporteigenschaften und eine verbesserte Freisetzungskinetik aufweisen sollen. Darüber hinaus soll die Zubereitung kein unangenehmes Klebrigkeitsgefühl auf der Haut hervorrufen.

Diese Aufgabe wird gelöst durch eine kosmetische oder dermatologische Zubereitung mit einem Wirkstoff zum Schutz oder zur Behandlung der Haut und/oder der Haare und mit einem Trägermaterial in oder an dem der Wirkstoff gebunden ist oder mit dem der Wirkstoff assoziiert ist, wobei das Trägermaterial einen Anteil an Chitosan aufweist und einen Anteil aus Polylactid, Polyglycolid und/oder Polylactid-Glycolid oder deren Derivaten aufweist, und wobei das Trägermaterial in Form von Partikeln mit einer mittleren Partikelgröße von 10 bis 1000 nm vorliegt.

Die Polymere Polylactid (PL), Polyglycolid (PG) und Polylactid-Glycolid (PLGA) sind biologisch resorbierbar und/oder biologisch abbaubar. Polylactide (Polymilchsäure) sind Polymere der Milchsäure mit D- und/oder L-Konfiguration. Polyglycolid (Polyglykolsäure) ist das Polymer der Glykolsäure. Polylactid-Glycolid ist ein Copolymer aus Milch- und Glykolsäure (auch: Poly(lactid-co-glycolid), wobei beide Moleküle in zufälliger Reihenfolge miteinander polymerisiert sind. Diese Polymere sind sehr gut verträglich, da sie im oder am Körper nicht akkumuliert werden, sondern abgebaut und/oder resorbiert und ausgeschieden oder verstoffwechselt werden. Da die Partikel gemäß der Erfindung als Hauptkomponenten des Trägermaterials biokompatible und biologisch abbaubare Polymere umfassen, wird eine Akkumulation der Zubereitung in oder am Körper durch die Bioabbaubarkeit der Polymere verhindert.

Die erfindungsgemäßen Partikel haben eine mittlere Partikelgröße von 10 bis 1000 nm. Vorzugweise haben die Partikel eine mittlere Partikelgröße von 10 bis 500 nm und besonders bevorzugt eine mittlere Partikelgröße von 50 bis 250 nm. Die erfindungsgemäßen Partikel dieser Größe werden im folgenden als Nanopartikel bezeichnet.

Zubereitungen mit erfindungsgemäßen Partikeln im Nanometerbereich haben Vorteile im Hinblick auf den Transport von Wirkstoffen/Schutzstoffen an bzw. in die Haut oder ihre Anhangorgane. Wegen ihrer geringen Größe ist es Nanopartikeln möglich, homogen und dicht gepackt in bzw. an der Haut oder ihren Anhangorganen verteilt zu werden. Nanopartikel werden im Vergleich zu größeren Partikeln schneller resorbiert und leichter abgebaut. Es wurde festgestellt, daß auch die kleinen Nanopartikel gemäß der Erfindung empfindliche Wirkstoffe effektiv schützen können.

Zu den weiteren Vorteilen der Erfindung zählt, daß die in den Zubereitungen enthaltenen Partikel die Wirkungsweise, Eindringtiefe, Schutzdauer, Verteilung, Stabilität, Applizierbarkeit und Bioverfügbarkeit des wenigstens einen Wirkstoffs, der chemisch und/oder physikalisch in oder an Partikeln gebunden oder mit diesen assoziiert ist, nachhaltig verbessern.

Das Freisetzungsprofil der verschiedenen Wirkstoffe kann mit Hilfe der Einstellung der Größe der Partikel beeinflußt werden. Bei Partikeln mit Anteilen an Lactid und Glycolid, wie z.B. Partikeln mit Polylactid-Glycolid oder Partikeln mit Polylactid und Polyglycolid, kann die Kinetik noch genauer dadurch definiert und eingestellt werden, daß das Verhältnis Glycolid zu Lactid variiert wird. Je höher der Anteil an Polyglycolid im Partikel ist, um so langsamer erfolgt eine Freisetzung der eingeschlossenen Wirkstoffe, da der enzymatische Abbau des Glycolids langsamer erfolgt als der des Lactids.

Der Anteil an Chitosan in dem Trägermaterial führt zu einer Modifikation der Oberfläche der-Trägerpartikel, indem positive Oberflächenladungen in die Trägerpartikel eingebracht werden. Da die Haut und deren Anhangorgane, wie z.B. die Haare eine negative Oberflächenladung aufweisen, ermöglichen die durch das Chitosan eingebrachten positiven Oberflächenladungen eine verbesserte Anhaftung der Partikel an der Haut bzw. deren Anhangorganen. Es wurde jedoch festgestellt, daß die Zubereitungen gemäß der Erfindung trotz ihres Anteils an Chitosan kein unangenehmes Klebrigkeitsgefühl auf der Haut erzeugen, was daran liegen mag, daß das Chitosan bei den erfindungsgemäßen Zubereitungen in die übrigen Bestandteile des Trägermaterials eingebettet vorliegt.

Darüber hinaus werden durch das Chitosan die Zubereitungen stabilisiert. Mit Hilfe verschiedener Anteile an Chitosan können die Größe und die Ladung der Partikel wie gewünscht eingestellt werden. Vorzugsweise weist das Trägermaterial der erfindungsgemäßen Zubereitung einen Anteil an Chitosan von 1 bis 50 Gew.-% auf. Besonders bevorzugt beträgt der Anteil an Chitosan von 5 bis 40 Gew.-%. Bei bevorzugten Ausführungsformen beträgt der Anteil an Chitosan von 10 bis 30 Gew.-%.

Darüber hinaus können die Partikel auch weitere positiv geladene Polymere enthalten. Die Beispiele für positiv geladene Polymere umfassen Polyethylenimin, Polylysin und deren Derivate oder Gemische davon. Mit Hilfe verschiedener Anteile an positiv geladenem Polymer können die Größe und die Ladung der Partikel wie gewünscht eingestellt werden. Vorzugsweise weist das Trägermaterial der erfindungsgemäßen Zubereitung insgesamt einen Anteil an positiv geladenem Polymer von 1 bis 70 Gew.-% auf. Besonders bevorzugt beträgt der Anteil von 5 bis 50 Gew.-% und noch bevorzugter von 10 bis 30 Gew.-%.

Das Gewichtsmittel des Molekulargewichtes der verwendeten Polymere beträgt vorzugsweise jeweils 10³ bis 2 x 10⁶ Dalton. Die Herstellungsverfahren für die verschiedenen Polymere sind dem Fachmann bekannt, und die meisten dieser Polymere sind von einer Reihe von Herstellern auch kommerziell erhältlich (vgl. Beispiele).

Durch Verwendung verschiedener bioabbaubarer Polymere in Kombination mit Chitosan und gegebenenfalls auch verschiedenen anderen Polymeren mit positiver Ladung kann die Oberflächenladung der Nanopartikel von positiven über neutrale bis hin zu negativen Werten eingestellt werden. Bei besonderen Ausführungsformen der Erfindung umfassen die Partikel zusätzlich auch Peptide zur Modifikation der Oberflächenladung der Partikel. Solche Oberflächenmodifikationen können den gezielten Transport zu einem Bestimmungsort ermöglichen bzw. erleichtern und somit unerwünschte Nebenwirkungen minimieren.

Die Oberflächenladung wird wiedergegeben durch das Zetapotential. Das Zetapotential (ζ-Potential) ist das elektrische Potential an der Abschergrenze eines bewegten Partikels in Suspension. Befinden sich geladene Partikel in Suspension, so wird deren Potential normalerweise durch Anlagerung von Ionen im Suspensionsmedium kompensiert, so daß das Partikel von außen betrachtet elektrisch neutral erscheint. Wird jedoch ein Partikel durch das Suspensionsmedium bewegt, wird ein Teil der angelagerten Ionen abgeschert und das Partikel erscheint nicht mehr elektrisch neutral, sondern besitzt an der Abschergrenze ein als Zetapotential bezeichnetes Potential. Das Zetapotential ist für das jeweilige Medium, in dem es gemessen wird, eine relative Meßgröße für das Oberflächenpotential und somit für die Ladung des Partikels. Für die Bestimmung des Zetapotentials werden die dem Fachmann bekannten Analysegeräte verwendet.

Das Zetapotential der erfindungsgemäßen Nanopartikel liegt vorzugsweise im Bereich von 0 mV bis +65 mV (gemessen in Wasser mit Zetamaster von Malvern). Vorzugsweise ist das Zetapotential der Partikel positiv. Besonders bevorzugt liegt das Zetapotential der erfindungsgemäßen Nanopartikel im Bereich von +20 mV bis +50 mV. Bei insbesondere bevorzugten Ausführungsformen liegt das Zetapotential im Bereich von +25 mV bis +45 mV. Noch bevorzugter liegt das Zetapotential im Bereich von +25 mV bis +35mV.

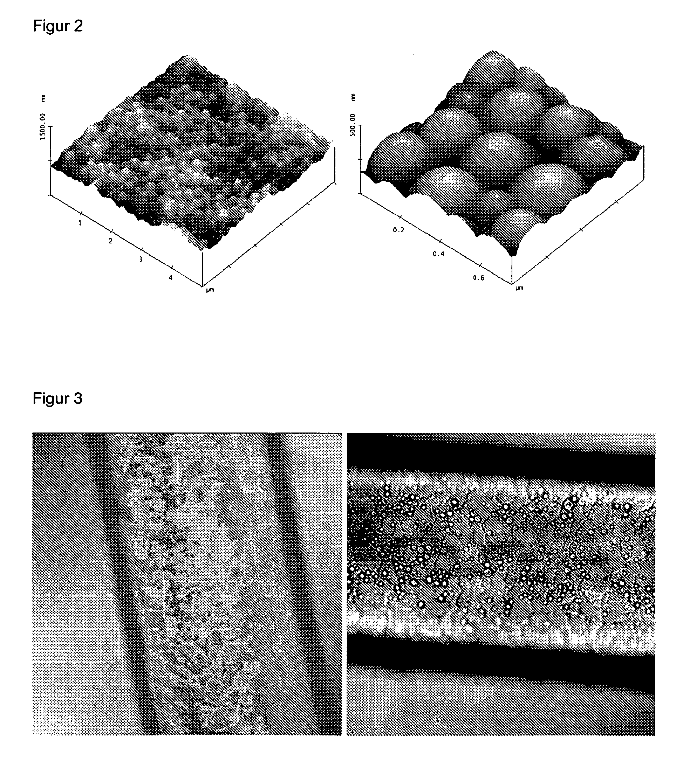

Die Form der Partikel ist vorzugsweise regelmäßig. Besonders bevorzugt sind die Partikel ideal sphärisch, d.h. vollständig rund, was mit dem Rasterkraftmikroskop (AFM - Atomic Force Microscopy) nachgewiesen werden kann (vgl. Figur 2). Bei alternativen Ausführungsformen ist es bevorzugt, daß die Partikelform unregelmäßig ist. Beispielsweise wird eine bessere und homogenere Oberflächenbedeckung mit im wesentlichen quaderförmigen Partikeln erreicht.

Bei bestimmten Ausführungsformen ist es bevorzugt, daß die Partikel porös sind. Die Porosität führt zu einer Vergrößerung der Partikeloberfläche, wodurch die Partikel mit mehr Wirk- oder Schutzstoff beladen werden können (Aktivkohle-Effekt). Bei alternativen Ausführungsformen sind die Partikel dagegen vorzugsweise nicht porös, was den Vorteil hat, daß die Beladung der Partikel mit Wirk- oder Schutzstoff und auch dessen Freisetzung besser kontrollierbar sind. Besonders bevorzugt sind Ausführungsformen, bei denen die Partikel ideal sphärisch sind und eine glatte, nicht poröse Oberfläche haben, was mit dem Rasterkraftmikroskop (AFM - Atomic Force Microscopy) nachgewiesen werden kann (vgl. Figur 2).

Vorzugsweise sind die Partikel monomodal verteilt, d.h. die überwiegende Mehrzahl (Gaußsche Verteilung) der auftretenden Partikel haben im wesentlichen die gleiche Form und Größe (Bestimmung der Form mit Rasterkraftmikroskop, Größenmessung mit Zetamaster). Vorzugsweise haben die Partikel eine sehr enge Größenverteilung und weisen daher eine geringe Polydispersität von vorzugsweise < 0.1 auf.

Der wenigstens eine Wirkstoff, der erfindungsgemäß chemisch und/oder physikalisch in oder an den Trägerpartikeln gebunden oder mit diesen assoziiert ist, kann ausgewählt sein unter Desodorantien bzw. Antitranspirantien, Insektenrepellentien, Vitaminen, Farbstoffen, Pigmenten, Fetten, Ölen, Wachsen, Lipiden oder Lipoiden oder anderen üblichen Bestandteilen einer kosmetischen oder dermatologischen Zubereitung.

Der wenigstens eine Wirkstoff, der erfindungsgemäß chemisch und/oder physikalisch in oder an den Trägerpartikeln gebunden oder mit diesen assoziiert ist, kann auch unter im pharmazeutischen Sinne wirksamen Stoffen ausgewählt sein, wie z.B. Corticoiden, Fungiziden, Antibiotika, nichtsteroidalen antiinflammatorisch und antientzündlich wirkenden Substanzen, Antirheumatika, Anästhetika, Antibiotika, Antiphlogistika sowie schmerzstillenden oder-lindernden Substanzen.

Der wenigstens eine Wirkstoff, der erfindungsgemäß chemisch und/oder physikalisch in oder an den Trägerpartikeln gebunden oder mit diesen assoziiert ist, kann auch ausgewählt sein unter Wirkstoffen, wie z.B. Coffein, Campher, Thymol, Hydrocortison bzw. dessen Derivaten, Vitaminen wie z.B. Ascorbinsäure und deren Derivaten, Vitaminen B und D, wobei die Vitamine B1, B12 und D1 besonders bevorzugt sind, ungesättigten bzw. essentiellen Fettsäuren. Der wenigstens eine Wirkstoff, kann aber auch ausgewählt sein unter Johannisbeerkernöl, Fischöl oder Ceramiden und deren Abkömmlingen.

Je nach Anwendung und Wirkstoff ist es bei bestimmten Ausführungsformen bevorzugt, wenn die Zubereitung nur einen Wirkstoff enthält. Bei alternativen Ausführungsformen ist es jedoch bevorzugt, wenn die Zubereitung mehrere Wirkstoffe umfaßt, wobei wenigstens einer der Wirkstoffe chemisch und/oder physikalisch in oder an den Trägerpartikeln gebunden oder mit diesen assoziiert ist. Besonders bevorzugt sind alle Wirkstoffe chemisch und/oder physikalisch in oder an die Trägerpartikel gebunden oder mit diesen assoziiert.

Bei den Ausführungsformen, bei denen der wenigstens eine Wirkstoff ein Desodorans bzw. Antitranspirans ist, kann dieses ausgewählt sein unter allen dem Fachmann bekannten, für kosmetische und dermatologische Zubereitungen zugelassenen Desodorantien bzw. Antitranspirantien, wie z. B. o-Chlorphenol , m-Chlorphenol, p-Chlorphenol, p-Chlor-m-xylenol, o-Chlorthymol, 2-Benzyl-4-chlorphenol (Chlorophen), 5,5'-Dichlor-2,2'-dihydroxydiphenylmethan (Dichlorophen), p-Hydroxybenzoesäure, 8-Hydroxychinolin, 2-Phenylphenol, 2,4,4"-Trichlor-2'-hydroxydiphenylether (Irgasan DP 300), Pyridinthiol-2, 2-Brom-2-nitropropandiol (Bronopol) und 1-(3-Chlorallyl)-3,5,7-triaza-I-azoniaadamantanchlorid (Dowicil 200). Besonders bevorzugte Ausführungsformen umfassen anorganische Bestandteile, wie Hectorit, Bentonit und Smectit etc. (R. Kieffer et al., "Analyse von Desodorantien in kosmetischen Präparaten", Fresenius Zeitschrift für Analytische Chemie, 1978, 293:135-137, W.P.Cheshire et al., "Disorders of Sweating", Seminars in Neurology 2003, 23/4: 399-406 und DE P4009347).

Bei besonders bevorzugten Ausführungsformen der Erfindung enthalten die Zubereitungen als Wirkstoffe eine oder mehrere Substanzen, welche die UVA- bzw. UVB-Strahlung absorbieren können. Vorzugsweise beträgt die Gesamtmenge solcher Sonnenschutzfilter von 0,1 bis 30 Gew.-% bezogen auf die Menge an Polymer in der Zubereitung. Diese speziellen Zubereitungen sind in der Lage, die Haut und ihre Anhangorgane, wie z.B. die Haare, vor schädlicher Sonnenstrahlung zu schützen.

Als UVB-Filter können gebräuchliche Sonnenschutzfilter, wie Benzylidencampher-Derivate, Aminobenzoesäure-Derivate, Ester der Zimtsäure, Ester der Salicylsäure, Ester der Benzalmalonsäure, Benzophenon-Derivate, Sulfonsäure-Derivate von Benzophenonen oder Benzylidencamphern, verwendet werden. Typische UVA-Filtersubstanzen stellen hierbei z.B. Derivate des Dibenzoylmethans dar. Die beschriebenen Sonnenschutzfilter sind nicht beschränkend hinsichtlich der erfindungsgemäßen Zubereitungen.

Bei bestimmten Ausführungsformen der Erfindung mit Sonnenschutzfilter ist es bevorzugt, daß diese Polylactid-Glycolid-Copolymere enthalten, bei denen das Verhältnis von Glycolat- zu Lactateinheiten im Polymer im Bereich von 50:50 bis 90:10 liegt. Vorzugsweise liegt das Verhältnis von Glycolat- zu Lactateinheiten bei einigen Ausführungsformen im Bereich von 70:30 bis 85:15.

Besonders bevorzugt enthalten die Zubereitungen gemäß der Erfindung einen oder mehrere anorganische Zusätze, wie z.B. Pigmente auf Basis von Metalloxiden (Zinkoxid, Titandioxid, Eisen(III)oxid, Zirkondioxid, Siliziumdioxid, Mangan(II)oxid, Aluminium(III)oxid oder Cer(III)oxid). Die vorgenannten Pigmente sind chemisch und/oder physikalisch in oder an den Trägerpartikeln gebunden oder mit diesen assoziiert oder auch lediglich in der die Partikel umgebenden Phase assoziiert und/oder dissoziiert. Die vorgenannten anorganischen Zusätze sind üblicherweise sowohl in amorpher sowie auch in regulärer kristalliner Form mit oder ohne hydrophobe Oberflächenmodifikation kommerziell erhältlich.

Bevorzugte Zubereitungen gemäß der Erfindung umfassen zusätzlich einen oder mehrere Hilfs- oder Zusatzstoffe, die auf die physikalischen Eigenschaften der Zubereitungen und deren Stabilität einwirken und/oder der Konservierung der Zubereitung dienen. Beispiele für solche Hilfs- oder Zusatzstoffe sind Tenside, Emulgatoren, Detergenzien, Kieselsäure, Wachse, Fette, Paraffine, Alkohole, Gelbildner, Gelatine, anorganische Zusätze, Konservierungsmittel, Bakterizide und Keimhemmer, Verdicker, Elektrolyte oder Komplexbildner.

Die bei bevorzugten Ausführungsformen der Erfindung verwendeten Hilfsstoffe zur Modifizierung der physikalisch-chemischen und biophysikalischen Eigenschaften umfassen Tenside, Stabilisatoren oder Hilfspolymere. Stabilisierend auf die erfindungsgemäßen Zubereitungen wirken unter anderem Zellulosederivate, Alginate, Polysaccharide oder deren Derivate, Carragene, Lipoide wie Cholesterol oder Phospholipide, Aerosil oder Bentonit und insbesondere Polyvinylalkohole mit einem Molekulargewicht von 10³ bis 10⁵ Dalton, vorzugsweise Polyvinylalkohole mit einem Molekulargewicht von 20.000 bis 40.000 Dalton.

Bei bevorzugten Ausführungsformen der Erfindung umfassen die Zubereitungen zusätzlich einen oder mehrere antiirritative oder antientzündliche Wirkstoffe, wie z.B. alpha-Oktadecylglycerylether, alpha-Hexadecylglycerylether, Bisabolol oder Panthenol.

Besonders bevorzugt umfassen die Zubereitungen gemäß der Erfindung ein oder mehrere Antioxidantien, wie z.B. Aminosäuren und deren Derivate, Carotine oder Carotinoide und deren Derivate, Liponsäure und deren Derivate, Folsäure, Tocopherole und deren Derivate, Vitamin A oder Vitamin E und deren Derivate. Vorzugsweise sind die Antioxidantien in der Zubereitung bezogen auf das Gesamtgewicht in einer Menge von 0,001 bis 30 Gew.-% enthalten, besonders bevorzugt in einer Menge von 1 bis 10 Gew.-%. Die Antioxidantien sind chemisch und/oder physikalisch in oder an den Trägerpartikeln gebunden oder mit diesen assoziiert oder auch lediglich in der die Partikel umgebenden Phase assoziiert und/oder dissoziiert.

Vorzugsweise werden die erfindungsgemäßen Nanopartikel mit Hilfe einer Dispersionstechnik, speziell mit dem nachfolgend beschriebenen speziellen "solvent evaporations"-Verfahren dargestellt. Hierdurch können auch Partikel mit einer besonders kleinen Größe von 50 bis 450 nm und einer sehr homogenen Verteilung hergestellt werden.

Bei dem bevorzugten Verfahren wird zunächst eine wässerige Lösung von Chitosan oder einem Derivat davon hergestellt. Bei einem besonders bevorzugten Verfahren wird zu dieser Lösung ein Anteil eines Polyvinylalkohols als Stabilisator zugegeben. Die Erfindung kann jedoch auch ohne Zugabe von Stabilisator ausgeführt werden. Parallel zur Herstellung der wässerigen Lösung von Chitosan wird eine Lösung von Polylactid, Polyglycolid und/oder Polylactid-Glycolid oder einem Derivat davon in einem organischen Lösungsmittel hergestellt. Vorzugsweise ist das organische Lösungsmittel nicht mischbar mit Wasser und leicht abzudampfen. Besonders bevorzugt ist das organische Lösungsmittel Ethylacetat. Bei bestimmten Ausführungsformen der Erfindung wird der Wirkstoff zum Schutz oder zur Behandlung der Haut und/oder der Haare bereits in dieser Phase in dem wässerigen Teil oder in dem organischen Teil gelöst, je nach seinen Löslichkeitseigenschaften.

Die wässerige Lösung und die Lösung in organischem Lösungsmittel werden vereinigt. Bei alternativen Ausführungsformen wird der Wirkstoff zum Schutz oder zur Behandlung der Haut und/oder der Haare erst in dieser Phase zugegeben.

Anschließend wird die Probe gerührt und letztlich mit einem Dispergiergerät homogenisiert. Bei alternativen Ausführungsformen wird der Wirkstoff zum Schutz oder zur Behandlung der Haut und/oder der Haare erst in dieser Phase zugegeben.

Im direkten Anschluß daran wird destilliertes Wasser hinzu gegeben. Bei alternativen Ausführungsformen wird der Wirkstoff zum Schutz oder zur Behandlung der Haut und/oder der Haare erst in dieser Phase zugegeben.

Im letzten Schritt wird das organische Lösungsmittel aus der Dispersion entfernt. Hierbei erfolgt die Partikelbildung. Besonders bevorzugt erfolgt die Entfernung des organischen Lösungsmittels durch Abdampfen des Lösungsmittels, vorzugsweise bei Raumtemperatur, besonders bevorzugt bei leicht erhöhter Temperatur. In der Regel entsteht hierbei eine milchig aussehende wässerige Suspension.

Alternativ können zur Herstellung der erfindungsgemäßen Partikel und Zubereitungen alle geeigneten Verfahren, die dem Fachmann bekannt sind, angewendet werden. Zu diesen Verfahren zählen die Sprühtrocknung, die Präzipitationstechnik, die Aussalztechnik und alle üblichen Fällungstechniken aus wässeriger oder organischer Phase. Vorzugsweise werden die Partikel durch Gefriertrocknung oder Sprühtrocknung als pulvrige Haufwerke dargestellt.

Die Zubereitungen gemäß der Erfindung können in alle für kosmetische und/oder dermatologische Zubereitungen geeigneten Formen eingearbeitet werden. Besonders bevorzugt liegen die Zubereitungen in Form von Suspensionen, Emulsionen (z.B. O/W, W/O, W/O/W etc.), Gelen, Salben, Suspensionssalben, Pasten und Cremes vor.

Die erfindungsgemäßen Nanopartikel zeichnen sich in allen gebräuchlichen kosmetischen und dermatologischen Zubereitungen durch eine hohe Stabilität aus, auch in Anwesenheit von Emulgatoren, Lipoiden, Salzen und Tensiden, aber auch bei Verwendung von Druckgasen. Besonders vorteilhaft gegenüber gebräuchlichen Trägersystemen ist ihre Anwendung in "Rinse-off'-Produkten, wie z.B. Shampoos und Duschbädern, was keine weiteren Anwendungen ausschließen soll.

Weitere Merkmale, Merkmalsgruppen und Ausführungsformen der vorliegenden Erfindung gehen aus den folgenden Beispielen und Figuren hervor.

Hierbei zeigen:
- Figur 1: eine schematische Darstellung einer Ausführungsform eines Herstellungsverfahrens für erfindungsgemäße Zubereitungen,
- Figur 2: eine Aufnahme von Partikeln gemäß der Erfindung durch ein Rasterkraftmikroskop,
- Figur 3: Aufnahmen der Anhaftung von Partikeln gemäß der Erfindung an ein menschliches Haar durch ein Rasterkraftmikroskop bzw. durch ein konfokales Laser-Scanning-Mikroskop,
- Figur 4: das Freisetzungsprofil einer Substanz aus erfindungsgemäßen Partikeln,
- Figur 5: Wirkstudie von erfindungsgemäßen Partikeln zum Schutz der Haut gegen Sonnen-Strahlung

### Beispiele

### I. Zubereitungen von erfindungsgemäßen Nanopartikeln mit UVA/B-Schutzfliter

### 1. Herstellung von erfindungsgemäßen Nanopartikeln mit dem UVA/B-Schutzfilter Eusolex^{®} 4360 unter Verwendung von Resomer^{®} RG 504S

25 mg Chitosan-Lactat (Rita Corporation) und 17,5 mg Mowiol 4-88 (Polyvinylalkohol der Firma Tel Hell & Co GmbH als Stabilisator) werden in einem Erlenmeyerkolben mit 2,5 ml bidestilliertem Wasser (Bidest-Anlage der Firma Fistreem) für 30 Minuten bei Raumtemperatur mit einem Magnetrührer (Firma IKA Werke GmbH & Co. KG, MSH basic yellowline) gerührt. Weiterhin wird eine Lösung mit 50 mg Poly-(DL-lactid-co-glycolid) (Resomer^{®} RG 504S von Boehringer Ingelheim Pharma GmbH & Co. KG) und 5 mg Eusolex^{®} 4360 (Sonnenschutzfilter von Merck KGaA) in 2,5 ml Ethylacetat unter Raumtemperaturbedingungen hergestellt.

In die wässerige Chitosan-Lösung wird unter ständigem Rühren mit einem Magnetrührer die Ethylacetat-Lösung hinzugetropft. Diese Dispersion wird für 1,5 Stunden in einem geschlossenen Gefäß bei Raumtemperatur gerührt. Anschließend wird die Probe für 10 Minuten einer Behandlung mit einem Dispergiergerät (Ultra-Turrax, IKA Labortechnik GmbH & Co. KG) unterzogen (13500 UpM), um die Dispersion zu homogenisieren. Im direkten Anschluß daran werden unter ständigem Rühren langsam und tropfenweise 22,5 ml bidestilliertes Wasser hinzu gegeben. Im letzten Schritt wird die Probe für 5 Stunden bei Raumtemperatur unter einem Abzug gerührt, um das Lösungsmittel Ethylacetat abzudampfen. Es entsteht eine milchig aussehende wässerige Suspension.

Die Größenbestimmung der Partikel mit Hilfe des "Zetasizer nano zs" (Malvern Instr.) ergab eine mittlere Partikelgröße von 401 nm (PDI 0,14). Das Zetapotential beträgt 9,9 mV (Malvern Zetamaster, in Wasser).

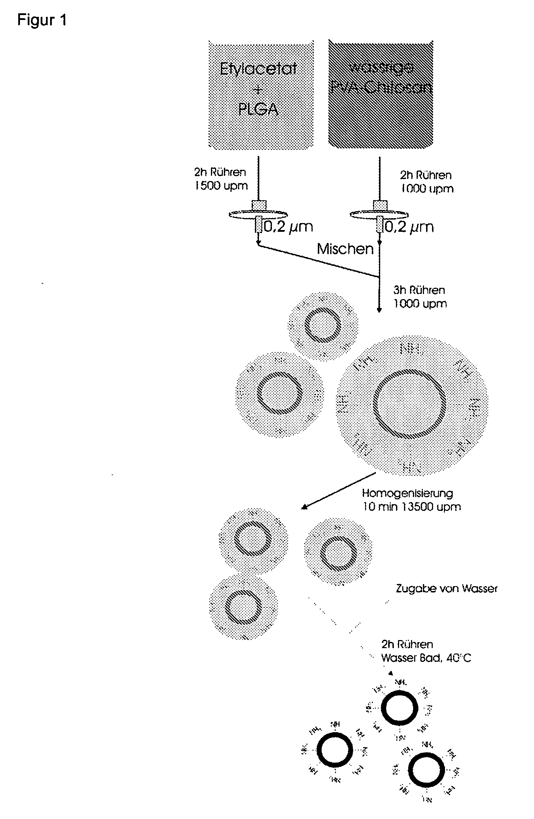

Figur 1 zeigt eine schematische Darstellung des Herstellungsverfahrens der PLGA/Chitosan-Nanopartikel von Beispiel I.1.

### 2. Herstellung von erfindungsgemäßen Nanopartikeln mit UVA/B-Schutzfilter unter Verwendung von Resomer^{®} RG 503H, Resomer^{®} RG 504S, Resomer^{®} RG 502S, PLGA (70:30) von Polyscience, PLGA (50:50) von Sigma und PLGA Purasorb^{®} (82:18), PLGA Purasorb^{®} (50:50)

Die Nanopartikel werden in der gleichen Weise hergestellt, wie für Beispiel 1.1. beschrieben, mit der Maßgabe, daß die in Tabelle 1 genannten Bestandteile in den dort angegebenen Konzentrationen vorliegen. Die in Gewichtsprozent angegebenen Konzentrationen beziehen sich auf den Anteil in der fertigen Gesamtzubereitung.

**Tabelle 1**

| | Chitosan [Gew.-%] | Eusolex^{®} [Gew.-%] | Partikelgröße [nm] | Zetapotential [mV] |
|---|---|---|---|---|
| Resomer^{®} RG 503H (50/50) [Glycolat/Lactat] von Boehringer 0.2 Gew.-% | 0,01% | 0,02% | 566 | 48 |
| | 0,02% | 0,02% | 558 | 54 |
| | 0,03% | 0,02% | 560 | 55 |
| | 0,04% | 0,02% | 563 | 55 |
| | 0,05% | 0,02% | 570 | 56 |
| | 0,06% | 0,02% | 640 | 60 |
| | 0,07% | 0,02% | 670 | 62 |
| | 0,08% | 0,02% | 689 | 62 |
| | 0,09% | 0,02% | 704 | 65 |
| | 0,1% | 0,02% | 710 | 65 |
| Resomer^{®} RG 504S von Boehringer 0.2 Gew.-% | 0,01% | 0,02% | 530 | 49 |
| | 0,02% | 0,02% | 533 | 49 |
| | 0,03% | 0,02% | 540 | 53 |
| | 0,04% | 0,02% | 554 | 55 |
| | 0,05% | 0,02% | 565 | 55 |
| | 0,06% | 0,02% | 618 | 58 |
| | 0,07% | 0,02% | 645 | 58 |
| | 0,08% | 0,02% | 663 | 55 |
| | 0,09% | 0,02% | 670 | 60 |
| | 0,1% | 0,02% | 690 | 60 |
| Resomer^{®} RG 502S von Boehringer 0.2 Gew.-% | 0,01% | 0,02% | 540 | 28 |
| | 0,02% | 0,02% | 545 | 34 |
| | 0,03% | 0,02% | 550 | 36 |
| | 0,04% | 0,02% | 580 | 36 |
| | 0,05% | 0,02% | 585 | 34 |
| | 0,06% | 0,02% | 644 | 38 |
| | 0,07% | 0,02% | 693 | 36 |
| | 0,08% | 0,02% | 700 | 38 |
| | 0,09% | 0,02% | 726 | 40 |
| | 0,1% | 0,02% | 740 | 42 |
| PLGA (70:30) [Glycolat/Lactat] von Polysience 0.2 Gew.-% | 0,01% | 0,02% | 334 | 36 |
| | 0,02% | 0,02% | 356 | 36 |
| | 0,03% | 0,02% | 343 | 37 |
| | 0,04% | 0,02% | 375 | 39 |
| | 0,05% | 0,02% | 411 | 37 |
| | 0,06% | 0,02% | 392 | 41 |
| | 0,07% | 0,02% | 424 | 39 |
| | 0,08% | 0,02% | 413 | 39 |
| | 0,09% | 0,02% | 454 | 40 |
| | 0,1% | 0,02% | 418 | 43 |
| PLGA (50:50) [Glycolat/Lactat] von Sigma 0.2 Gew.-% | 0,01% | 0,02% | 310 | 34 |
| | 0,02% | 0,02% | 324 | 32 |
| | 0,03% | 0,02% | 325 | 34 |
| | 0,04% | 0,02% | 333 | 36 |
| | 0,05% | 0,02% | 356 | 35 |
| | 0,06% | 0,02% | 371 | 38 |
| | 0,07% | 0,02% | 368 | 37 |
| | 0,08% | 0,02% | 379 | 39 |
| | 0,09% | 0,02% | 396 | 42 |
| | 0,1% | 0,02% | 385 | 42 |
| PLGA Purasorb^{®} (82:18) [Glycolat/Lactat] von Purac | 0,01% | 0,02% | 293 | 34 |
| 0.2 Gew.-% | | | | |
| | 0,02% | 0,02% | 295 | 43 |
| | 0,03% | 0,02% | 312 | 42 |
| | 0,04% | 0,02% | 310 | 44 |
| | 0,05% | 0,02% | 335 | 43 |
| | 0,06% | 0,02% | 346 | 46 |
| | 0,07% | 0,02% | 370 | 46 |
| | 0,08% | 0,02% | 367 | 46 |
| | 0,09% | 0,02% | 372 | 45 |
| | 0,1% | 0,02% | 380 | 48 |
| PLGA Purasorb^{®} (50:50) [Glycolat/Lactat] von Purac 0.2 Gew.-% | 0,01% | 0,02% | 187 | 27 |
| | 0,02% | 0,02% | 189 | 30 |
| | 0,03% | 0,02% | 189 | 29 |
| | 0,04% | 0,02% | 193 | 29 |
| | 0,05% | 0,02% | 199 | 31 |
| | 0,06% | 0,02% | 198 | 29 |
| | 0,07% | 0,02% | 204 | 35 |
| | 0,08% | 0,02% | 209 | 29 |
| | 0,09% | 0,02% | 210 | 28 |
| | 0,1% | 0,02% | 212 | 30 |

Figur 2 ist eine Aufnahme durch ein Rasterkraftmikroskop von Nanopartikeln der Zusammensetzung PLGA Purasorb (82:18) mit 0,02 Gew.-% Chitosan und 0,02 Gew.-% Eusolex 3640. Die Nanopartikel besitzen eine ideal sphärische Gestalt mit einer glatten nicht porösen Oberfläche.

### 3. Herstellung von erfindungsgemäßen Nanopartikeln mit dem Antioxidans

Die Nanopartikel werden in der gleichen Weise hergestellt, wie für Beispiel I.1. beschrieben, mit der Maßgabe, daß die in Tabelle 2 genannten Bestandteile in den dort angegebenen Konzentrationen vorliegen. Die in Gewichtsprozent angegebenen Konzentrationen beziehen sich auf den Anteil in der fertigen Gesamtzubereitung.

**Tabelle 2**

| PLGA | Chitosan [Gew.-%] | Tocopherol [Gew.-%] | Partikelgröße [nm] | Zetapotential [mV] |
|---|---|---|---|---|
| PLGA Purasorb^{®} (82:18) von Purac 0.2 Gew.-% | 0,01% | 0,02% | 220 | 27 |
| | 0,02% | 0,02% | 223 | 29 |
| | 0,03% | 0,02% | 225 | 26 |
| | 0,04% | 0,02% | 228 | 31 |
| | 0,05% | 0,02% | 218 | 25 |
| | 0,06% | 0,02% | 229 | 29 |
| | 0,07% | 0,02% | 217 | 24 |
| | 0,08% | 0,02% | 226 | 27 |
| | 0,09% | 0,02% | 223 | 24 |
| | 0,1% | 0,02% | 231 | 25 |
| | | | | |

| PLGA | Chitosan [Gew.-%] | Retinol [Gew.-%] | Partikelgröße [nm] | Zetapotential [mV] |
|---|---|---|---|---|
| PLGA Purasorb^{®} (82:18) von Purac 0.2 Gew.-% | 0,01% | 0,002% | 195 | 31 |
| | 0,02% | 0,002% | 196 | 28 |
| | 0,03% | 0,002% | 194 | 32 |
| | 0,04% | 0,002% | 201 | 32 |
| | 0,05% | 0,002% | 198 | 31 |
| | 0,06% | 0,002% | 210 | 35 |
| | 0,07% | 0,002% | 203 | 33 |
| | 0,08% | 0,002% | 202 | 31 |
| | 0,09% | 0,002% | 208 | 36 |
| | 0,1% | 0,002% | 209 | 29 |

### II. Anwendung der erfindungsgemäßen Nanopartikel

### 1. Anhaftung der erfindungsgemäßen Nanopartikel an menschlichen Haaren

Nanopartikel aus PLGA (82/18) Purac mit 0,02 Gew.-% Chitosan und 0,02 Gew.-% Eusolex (bezogen auf die Gesamtzubereitung) wurden unter Verwendung von mit zusätzlichem FITC (Fluoresceinisothiocyanat) markiertem Chitosan wie oben beschrieben hergestellt. Die Fluoreszenzmarkierung diente der Visualisierung des Anhaftvermögens der Nanopartikel. Eine Strähne menschlichen Kopfhaares wurde für 10 Minuten vollständig in der Partikelsuspension inkubiert, anschließend 3 mal intensiv mit Wasser gewaschen und getrocknet. Die Haare wurden danach auf einem Objektträger mit Hilfe von Fluorsave (Firma Calbiochem) fixiert und unter einem konfokalen Laser-Scanning-Mikroskop der Firma Zeiss betrachtet (Excitationswellenlänge: 488 nm, Emissionswellenlänge: 518 nm). Eine homogene Verteilung und das stabile Anhaften der Partikel an den Haaren konnte so nachgewiesen werden.

Figur 3 zeigt Aufnahmen mit konfokaler Laser-Scanning-Mikroskopie (links) und Rasterkraftmikroskopie (rechts) der Anhaftung von erfindungsgemäßen Nanopartikeln mit der Zusammensetzung PLGA (82/18) mit 0,02 Gew. % Chitosan und 0,02 Gew. % Eusolex an einem menschlichen Haar. Bei der Aufnahme mit dem konfokalen Laser-Scanning-Mikroskop sind die hellen Stellen auf dem abgebildeten Haar, die Bereiche in denen sich die Partikel mit fluoreszenzmarkiertem Chitosan an das Haar angeheftet haben.

### 2. Stabilität und Freisetzung des Wirkstoffes

Die Veränderung der Wirkstoffkonzentration wurde nach Anhaftung der Nanopartikel (PLGA (82:18) Purac mit 0,02 Gew.-% Chitosan und 0,02 Gew.-% Eusolex, ohne Fluoreszenzfarbstoff) an die Haare durch Freisetzungsstudien photometrisch ermittelt. Die maximale Freisetzung entspricht 66 µg / ml = 100%.

In Abhängigkeit von der verwendeten Polymerzusammensetzung ist die Freisetzung im Zeitintervall von 24 h bis 28 Tage frei einstellbar.

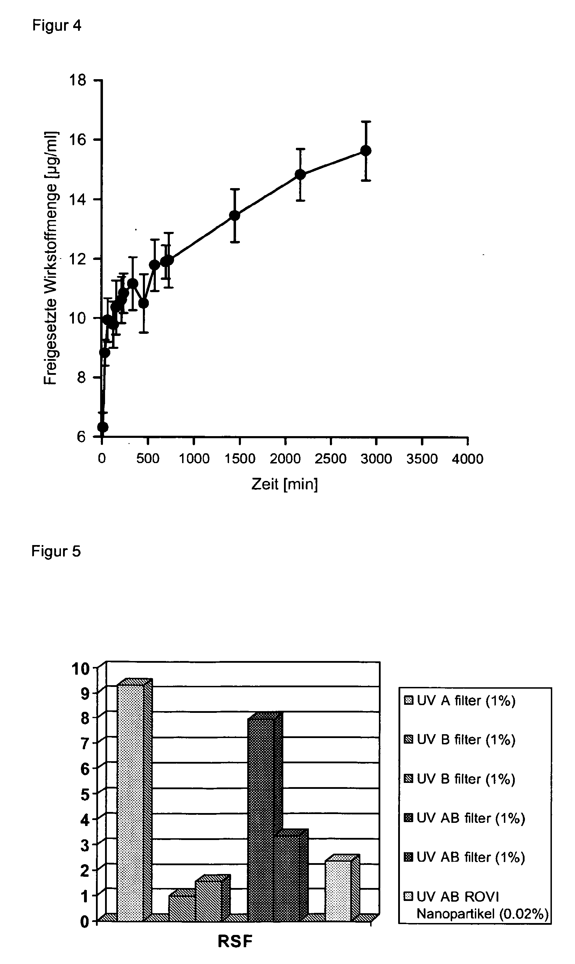

Figur 4 zeigt die Freisetzungskinetik des Eusolex aus den Nanopartikeln

### 3. Adsorption der erfindungsgemäßen Nanopartikel an die Haut und hierdurch erlangter Schutz der Haut gegen Sonnenstrahlung

Ähnlich dem SPF (Sun Protection Faktor) wird bei der Bestimmung des RSF (Radical Skin Protection Factor) die Wirkung der UV-Strahlung auf die Haut gemessen. Als Meßgröße bzw. Indikator im Vergleich zwischen unbestrahlter und bestrahlter Haut wird nicht das als biologischer Endpunkt bezeichnete Erythem verwendet, sondern es wird die Menge und Art an UV-generierten freien Radikalen (ROS, reactive oxygen species) in der Haut bestimmt. Diese freien Radikale sind proportional zur UV-Dosis und ihre Verteilung in den einzelnen Hautschichten (Epidermis, Dermis) wird maßgeblich von der Wellenlänge der eindringenden Photonen bestimmt.

Die Bestimmung des RSF wird zweckmäßigerweise als ex-vivo-Methode an Hautbiopsien durchgeführt. Hautbiopsien werden mit einem Radikalindikator (Nitroxyl-Verbindung) markiert. Die durch die UV-Strahlung (Sonnensimulator) in der Haut generierten freien Radikale (ROS) reagieren mit dem Radikalindikator, dessen Menge und Aktivität mit einem ESR-Spektrometer (Elektronenspinresonanzspektrometer) gemessen wird. Die Bildung der durch UVA und UVB induzierten freien Radikale ist ein Prozeß, der sich in der epidermalen und dermalen Schicht der Haut abspielt. Die Wirkung der UVB-Strahlung beschränkt sich, bedingt durch die geringe Eindringtiefe, hauptsächlich auf die Epidermis. Der Radikalindikator ist hydrophil und weist demzufolge in der Dermis und Epidermis eine morphologisch bedingte Gleichverteilung auf. Der Radikalindikator selbst ist fotostabil und wird in wässeriger Lösung nicht durch UV abgebaut.

Die in der Haut induzierten freien Radikale/ROS oxidieren das Molekül der Nitroxyl-Verbindung und können so mit der ESR-Spektroskopie nachgewiesen werden. Über diesen Effekt kann die UV-schützende Wirkung von UV-Filtern getestet werden. Während das UVA ausschließlich freie Radikale/ROS in der Haut generiert, kann das UVB (insbesondere das kurzwellige UVB) auch zu direkten Schäden an der Erbinformation (DNA) der Zelle führen. Die RSF-Methode ist durch ihre direkte Radikalmessung besonders sensitiv gegenüber der Wirkung von UVA-Strahlung und kann dementsprechend die Wirkung von UVA-Filtern eindeutig charakterisieren.

Im Gegensatz zum SPF werden mit der Bestimmung der RSF sowohl die Schäden, die durch UVA- und UVB-Strahlung erzeugt werden, erfaßt.

Es wurden auf die Haut folgende Formulierungen in einer Menge von 2 mg/cm² aufgetragen:
- Wässerige Lösung mit UV-Nanopartikeln (0.2 Gew.-% PLGA (82/18) von Purac; 0.02 Gew.-% Chitosan; 0.02 Gew.-% Eusolex 4360)
- Ethanolisch-wässerige Lösung mit jeweils 1 % von Filter

Figur 5 zeigt den RSF (Radikal Skin Protection Factor) unterschiedlicher Filter, die zum Schutz der Haut gegen Sonnenlicht eingesetzt wurden. Obwohl bei der Zubereitung mit Nanopartikeln gemäß der Erfindung (UV AB ROVI Nanopartikel) eine deutlich geringere Menge an UV-Filter (0.02%) auf die Haut aufgetragen wurde, nämlich nur 1/50 der Filtermenge, die bei den Kontrollen eingesetzt wurde, konnte eine sehr effiziente Schutzschicht auf der Haut gebildet werden, die 50% der schädigenden Strahlung abwenden konnte. Dies macht es möglich, daß nur sehr wenig UV-Filtersubstanz in Sonnenschutzzubereitungen verwendet werden muß. Dies ist wünschenswert, da die Filtersubstanzen teilweise unter dem Verdacht stehen, hormonähnliche Wirkung zu haben, was sich bei hoher Dosierung für den Anwender nachteilig auswirken könnte.

## Patentansprüche

1. Kosmetische oder dermatologische Zubereitung mit einem Wirkstoff zum Schutz oder zur Behandlung der Haut und/oder der Haare und mit einem Trägermaterial in oder an dem der Wirkstoff gebunden ist oder mit dem der Wirkstoff assoziiert ist, wobei das Trägermaterial einen Anteil an Chitosan aufweist und einen Anteil aus Polylactid, Polyglycolid und/oder Polylactid-Glycolid oder deren Derivaten aufweist, und wobei das Trägermaterial in Form von Partikeln mit einer mittleren Partikelgröße von 10 bis 1000 nm vorliegt.

2. Zubereitung nach Anspruch 1, **dadurch gekennzeichnet, daß** der Anteil des Chitosan im Trägermaterial von 1 bis 50 Gew.-% beträgt.

3. Zubereitung nach einem der Ansprüche 1 oder 2, **dadurch gekennzeichnet, daß** das Zetapotential der Partikel im Bereich von 0 mV bis +65 mV liegt, vorzugsweise im Bereich von +20 mV bis +50 mV liegt und besonders bevorzugt im Bereich von +25 mV bis +45 mV liegt.

4. Zubereitung nach einem der Ansprüche 1 bis 3, **dadurch gekennzeichnet, daß** die mittlere Partikelgröße der Partikel von 10 bis 500 nm beträgt, vorzugsweise von 50 bis 250 nm beträgt.

5. Zubereitung nach einem der Ansprüche 1 bis 4, **dadurch gekennzeichnet, daß** die Partikel ideal sphärisch sind.

6. Zubereitung nach einem der Ansprüche 1 bis 5, **dadurch gekennzeichnet, daß** das Trägermaterial weitere kationische biologisch abbaubare Polymere umfaßt, vorzugsweise Polylysin.

7. Zubereitung nach einem der Ansprüche 1 bis 6, **dadurch gekennzeichnet, daß** der wenigstens eine Wirkstoff ausgewählt ist unter Desodorantien, Antitranspirantien, Insektenrepellentien, Vitaminen, Farbstoffen, Pigmenten, Fetten, Ölen, Wachsen, Lipiden oder Lipoiden, Corticoiden, Fungiziden, Antibiotika, nichtsteroidalen antiinflammatorischen und antientzündlich wirkenden Substanzen, Antirheumatika, Anästhetika, Antiphlogistika sowie schmerzstillenden oder -lindernden Substanzen, Coffein, Campher, Thymol, Hydrocortison oder dessen Derivaten, Vitaminen, ungesättigten oder essentiellen Fettsäuren, Johannisbeerkernöl, Fischöl oder Ceramiden und deren Derivaten.

8. Zubereitung nach einem der Ansprüche 1 bis 7, **dadurch gekennzeichnet, daß** der wenigstens eine Wirkstoff ausgewählt ist unter Substanzen, die die UVA- und/oder UVB-Strahlung absorbieren können.

9. Zubereitung nach einem der Ansprüche 1 bis 8, **dadurch gekennzeichnet, daß** sie wenigstens einen Hilfs- oder Zusatzstoff zur Modifikation der physikalischen Eigenschaften und/oder der Stabilität der Zubereitung und/oder zur Konservierung der Zubereitung umfaßt, wobei der Hilfs- oder Zusatzstoff ausgewählt ist unter Tensiden, Emulgatoren, Detergenzien, Kieselsäuren, Wachsen, Fetten, Paraffinen, Alkoholen, Gelbildnern, Gelatine, anorganischen Zusätzen, Konservierungsmitteln, Bakteriziden und Keimhemmern, Verdickern, Elektrolyten und Komplexbildnern.

10. Zubereitung nach einem der Ansprüche 1 bis 9, **dadurch gekennzeichnet, daß** sie einen antiirritativen oder antientzündlichen Wirkstoff umfaßt, wobei dieser Wirkstoff vorzugsweise ausgewählt ist unter alpha-Oktadecylglycerylether, alpha-Hexadecylglycerylether, Bisabolol und Panthenol oder Gemischen davon.

11. Zubereitung nach einem der Ansprüche 1 bis 10, **dadurch gekennzeichnet, daß** sie ein Antioxidans umfaßt, wobei das Antioxidans vorzugsweise ausgewählt ist unter Aminosäuren, Carotinen, Carotinoiden, Liponsäure, Folsäure, Tocopherolen, Vitamin A, Vitamin E und deren Derivaten oder Gemischen davon.

12. Zubereitung nach einem der Ansprüche 1 bis 11, **dadurch gekennzeichnet, daß** sie in eine Formulierung eingearbeitet ist, die ausgewählt ist unter einer Suspension, einer Emulsion, einem Gel, einer Salbe, einer Suspensionssalbe, einer Paste oder einer Creme, einem Shampoo oder einer Waschlösung.

## Claims

1. Cosmetic or dermatological preparation comprising an active agent for protecting or treating skin and/or hair and comprising a carrier material in or on which the active agent is bound to or to which the active agent is associated with, wherein the carrier material comprises an amount of chitosan and an amount of polylactide, polyglycolide, and/or polylactide-glycolide or derivatives thereof and wherein the carrier material is present in form of particles with an average particle size of 10 to 1000 nm.

2. Preparation according to claim 1, **characterized in that** the amount of chitosan in the carrier material is 1 to 50 % by weight.

3. Preparation according to one of claims 1 or 2, **characterized in that** the zeta potential of the particles is in the range of 0 mV to +65 mV, preferably in the range of +20 mV to +50 mV und particularly preferably in the range of +25 mV to +45 mV.

4. Preparation according to one of claims 1 to 3, **characterized in that** the average particle size of the particles is from 10 to 500 nm, preferably from 50 to 250 nm.

5. Preparation according to one of claims 1 to 4, **characterized in that** the particles are ideally spherical.

6. Preparation according to one of claims 1 to 5, **characterized in that** the carrier material comprises further cationic biodegradable polymers, preferably polylysine.

7. Preparation according to one of claims 1 to 6, **characterized in that** the at least one active agent is selected from deodorants, antiperspirants, insect repellents, vitamins, colorants, pigments, fats, oils, waxes, lipids or lipoids, corticoids, fungicides, antibiotics, non-steroidal anti-inflammatory and anti-phlogistic acting substances, anti-rheumatics, anaesthetics, antiphlogistics as well as analgesic or pain-relieving substances, caffeine, camphor, thymol, hydrocortisone or derivatives thereof, vitamins, unsaturated or essential fatty acids, blackcurrant seed oil, fish oil or ceramids and derivatives thereof.

8. Preparation according to one of claims 1 to 7, **characterized in that** the at least one active agent is selected from substances, which can absorb UVA and/or UVB radiation.

9. Preparation according to one of claims 1 to 8, **characterized in that** it comprises at least one adjuvant or additive for modifying the physical characteristics and/or the stability of the preparation and/or for conserving of the preparation, wherein the adjuvant or additive is selected from tensides, emulsifiers, detergents, silicic acids, waxes, fats, paraffins, alcohols, gelling agents, gelatine, inorganic additives, preservatives, bacterizides and anti-germinating agents, thickeners, electrolytes and complexing agents.

10. Preparation according to one of claims 1 to 9, **characterized in that** it comprises an anti-irritant or anti-inflammatory substance, wherein this substance is preferably selected from alpha-octadecylglycerylether, alpha-hexadecylglycerylether, bisabolol and pantheol or mixtures thereof.

11. Preparation according to one of claims 1 to 10, **characterized in that** it comprises an antioxidant, wherein the antioxidant is preferably selected from amino acids, carotenes, carotenoids, lipoic acid, folic acid, tocopherols, vitamin A, vitamin E and derivatives or mixtures thereof.

12. Preparation according to one of claims 1 to 11, **characterized in that** it is incorporated in a formulation, which is selected from a suspension, an emulsion, a gel, an ointment, a suspension ointment, a paste or a cream, a shampoo or a washing solution.

## Revendications

1. Préparation cosmétique ou dermatologique avec un principe actif pour la protection ou le traitement de la peau et/ou des cheveux et avec un matériau porteur dans ou sur lequel le principe actif est amalgamé ou auquel le principe actif est combiné, le matériau porteur présentant une proportion de chitosan et une proportion de polyactide, polyglycolide et/ou polyactide-polyglycolide ou leurs dérivés, et le matériau porteur étant présent sous forme de particules d'une taille allant de 10 à 1 000 nm.

2. Préparation selon la revendication 1, **caractérisée en ce que** la proportion de chitosan dans le matériau porteur se situe entre 1 et 50 % de son poids.

3. Préparation selon la revendication 1 ou 2, **caractérisée en ce que** le potentiel zêta des particules se situe entre 0 mV et + 65 mV, de préférence entre + 20 mV et + 50 mV et en particulier de préférence entre + 25 mV et + 45 mV.

4. Préparation selon l'une des revendications 1 à 3, **caractérisée en ce que** la taille moyenne des particules se situe entre 10 et 500 nm, de préférence entre 50 et 250 mm.

5. Préparation selon l'une des revendications 1 à 4, **caractérisée en ce que** les particules sont des sphères idéales.

6. Préparation selon l'une des revendications 1 à 5, **caractérisée en ce que** le matériau porteur comprend d'autres polymères cationiques biologiquement dégradables, de préférence une polylysine.

7. Préparation selon l'une des revendications 1 à 6, **caractérisée en ce qu'**au moins un principe actif est choisi parmi des déodorants, anti-transpirants, répulsifs pour insectes, vitamines, colorants, pigments, graisses, huiles, cires, lipides ou lipoïdes, corticoïdes, fongicides, antibiotiques, non stéroïdiens, anti-inflammatoires et substances à action anti-inflammatoire, anti-rhumatismaux, anesthésiques, antiphlogistiques, ainsi que parmi des substances antalgiques ou analgésiques, caféine, camphre, thymol, hydrocortisone ou ses dérivés, vitamines, aides gras non saturés ou essentiels, huile de pépins de groseilles, huile de poisson ou céramides et leur dérivés.

8. Préparation selon l'une des revendications 1 à 7, **caractérisée en ce qu'**au moins un principe actif est choisi parmi des substances capables d'absorber le rayonnement UVA et/ou UVB.

9. Préparation selon l'une des revendications 1 à 8, **caractérisée en ce qu'**elle comprend au moins un excipient ou adjuvant pour modifier les propriétés physiques et/ou la stabilité de la préparation et/ou pour conserver la préparation, l'excipient ou adjuvant étant choisi parmi des tensides, émulsifiants, détergents, silices, cires, graisses, paraffines, alcools, gélifiants, gélatines, additifs anorganiques, agents de conservation, bactéricides et germicides, épaississants, électrolytes et agents complexants.

10. Préparation selon l'une des revendications 1 à 9, **caractérisée en ce qu'**elle comprend un principe actif anti-irritant ou anti-inflammatoire, ce principe actif étant de préférence choisi parmi des éthers de glycéride octadécyle, éthers de glycéride alpha-hexadécyle, bisabolol et panthénol ou des mélanges de ceux-ci.

11. Préparation selon l'une des revendications 1 à 10, **caractérisée en ce qu'**elle comprend un anti-oxydant, l'anti-oxydant étant de préférence choisi parmi des aminoacides, carotènes, caroténoïdes, acides lipoïques, acides foliques, tocophéroles, vitamine A, vitamine E et leurs dérivés ou des mélanges de ceux-ci.

12. Préparation selon l'une des revendications 1 à 11, **caractérisée en ce qu'**elle est insérée dans une formulation, laquelle est choisie parmi une suspension, une émulsion, un gel, un onguent, une pommade, une pâte ou une crème, un shampoing ou une solution de lavage.
